(19) Europäisches Patentamt
European Patent Office
Office européen des brevets

(11) **EP 2 671 594 A1**

(12) **EUROPEAN PATENT APPLICATION**
published in accordance with Art. 153(4) EPC

(43) Date of publication:
**11.12.2013 Bulletin 2013/50**

(51) Int Cl.:
***A61K 47/12*** (2006.01)    ***A61K 9/70*** (2006.01)
***A61K 31/167*** (2006.01)    ***A61K 47/32*** (2006.01)

(21) Application number: **12742503.1**

(22) Date of filing: **01.02.2012**

(86) International application number:
**PCT/JP2012/052309**

(87) International publication number:
**WO 2012/105621 (09.08.2012 Gazette 2012/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.02.2011 JP 2011021203**

(71) Applicant: **Nitto Denko Corporation Osaka 567-8680 (JP)**

(72) Inventors:
• **OKADA, Katsuhiro**
  **Ibaraki-shi**
  **Osaka 567-8680 (JP)**

• **NISHIMURA, Masato**
  **Ibaraki-shi**
  **Osaka 567-8680 (JP)**
• **KAWAHARADA, Yuji**
  **Ibaraki-shi**
  **Osaka 567-8680 (JP)**
• **OKADA, Yasuaki**
  **Ibaraki-shi**
  **Osaka 567-8680 (JP)**

(74) Representative: **Gill, Stephen Charles**
  **Mewburn Ellis LLP**
  **33 Gutter Lane**
  **London**
  **EC2V 8AS (GB)**

(54) **PRODUCTION METHOD FOR ADHESIVE PATCH**

(57) The problem of the present invention is to minimize the influence of lactic acid on the property of an adhesive layer, and provide a production method of a patch preparation, which is capable of affording a patch preparation superior in the property of an adhesive layer.

A method of producing a patch preparation comprising (a): a step of forming an adhesive layer containing a drug (excluding 2-(4-ethyl-1-piperazinyl)-4-(4-fluorophenyl)-5,6,7,8,9,10-hexahydrocycloocta[b]pyridine and a physiologically acceptable acid addition salt thereof), an acrylic polymer and an organic liquid component compatible with the acrylic polymer, but without containing lactic acid, and (b): a step of adding lactic acid to the aforementioned adhesive layer.

EP 2 671 594 A1

**Description**

Technical Field

The present invention relates to a production method of a patch preparation having an adhesive layer containing a drug excluding 2-(4-ethyl-1-piperazinyl)-4-(4-fluorophenyl)-5,6,7,8,9,10-hexahydrocycloocta[b]pyridine and a physiologically acceptable salt thereof, and lactic acid.

Background Art

A transdermal administration of a drug can avoid first pass effect of the liver since the drug can be directly absorbed from the capillary of the skin surface. In transdermal administration, moreover, since a drug is released in a sustained manner, side effects caused by absorption of a large amount of the drug in a short time can be reduced. Therefore, transdermal administration is one of the effective means of drug administration.
Transdermal absorption preparation containing a drug has already been known (patent document 1). Patent document 1 also discloses that lactic acid increases transdermal absorbability of the drug.
On the other hand, patent document 2 describes a patch preparation containing lactic acid, and describes that lactic acid increases transdermal absorbability of a drug. However, patent documents 1, 2 both do not refer to the influence of lactic acid on the property of an adhesive layer.

[Document List]

[patent documents]

patent document 1: WO2007/142295
patent document 2: WO96/16642

SUMMARY OF THE INVENTION

Problems to be Solved by the Invention

During the course of consideration by the present inventors, it has been found that the influence of lactic acid on the property of an adhesive layer is not small, and countermeasures should be taken. Therefore, the problem of the present invention is to minimize the influence of lactic acid on the property of an adhesive layer, and provide a production method of a patch preparation, which is capable of affording a patch preparation superior in the property of an adhesive layer.

Means of Solving the Problems

The present inventors have conducted intensive studies in an attempt to solve the aforementioned problems and found that lactic acid can be contained in an adhesive layer by forming an adhesive layer containing a drug but without containing lactic acid, and applying lactic acid to an adhesive surface of the adhesive layer, and that a transdermal drug absorption-promoting effect by lactic acid can be sufficiently obtained also in the thus-obtained preparation, based on which findings they have conducted further studies and completed the present invention.
Accordingly, the present invention provides the following.

[1] A method of producing a patch preparation comprising, in an adhesive layer, a drug (excluding 2-(4-ethyl-1-piperazinyl)-4-(4-fluorophenyl)-5,6,7,8,9,10-hexahydrocycloocta[b]pyridine and a physiologically acceptable acid addition salt thereof), and lactic acid, comprising

(a): a step of forming an adhesive layer containing a drug, an acrylic polymer and an organic liquid component compatible with the acrylic polymer, but without containing lactic acid, and
(b): a step of adding lactic acid to the aforementioned adhesive layer.

[2] The method of the above-mentioned [1], wherein a crosslinked adhesive layer is formed in step (a).
[3] The method of the above-mentioned [1] or [2], wherein the adhesive layer is formed on one surface of a support or a release liner in step (a).

[4] The method of any one of the above-mentioned [1] to [3], wherein the lactic acid is contained in the adhesive layer by immersing the adhesive layer in a lactic acid solution obtained by dissolving lactic acid in an organic solvent in step (b).

[5] The method of the above-mentioned [4], wherein the adhesive layer is impregnated with the lactic acid solution by applying the lactic acid solution to a surface of the adhesive layer and leaving the adhesive layer at 5 - 40°C for a given time.

[6] The method of the above-mentioned [4] or [5], further comprising, after step (b), (c): a step of evaporating an organic solvent derived from the lactic acid solution contained in the adhesive layer.

Effect of the Invention

According to the production method of the present invention, since lactic acid is added later to an adhesive layer without containing lactic acid, an influence of lactic acid on the property of the adhesive layer can be reduced. Particularly, when a patch preparation containing a drug and lactic acid in a crosslinked adhesive layer is to be produced, an adverse influence of lactic acid on the crosslinking reaction of the adhesive layer can be suppressed. Therefore, a patch preparation having an improved holding power after adhesion to the skin (i.e., cohesive force of the adhesive layer after adhesion), while maintaining a transdermal drug absorption-promoting effect of lactic acid, can be produced. Description of Embodiments

The drug relating to the present invention is not particularly limited as long as 2-(4-ethyl-1-piperazinyl)-4-(4-fluorophenyl)-5,6,7,8,9,10-hexahydrocycloocta[b]pyridine and a physiologically acceptable salt thereof are excluded, and a transdermally absorbable drug, which can be administered to mammals such as human and the like through the skin, is preferable. Specific examples of such drug include general anesthetics, hypnotic sedatives, antiepileptic drugs, antipyretic analgesic antiphlogistic drugs, anti-vertiginous drugs, psychoneurotic drugs, central neurological drug, antidementia, local anesthetics, skeletal muscle relaxants, autonomic drugs, antispasmodic drugs, anti-parkinson drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for purulent diseases, analgesic-antipruritic-styptic-antiinflammatory drugs, drugs for parasitic skin diseases, hemostatic drugs, gout treatment drugs, drugs for diabetes, anti-malignant tumor agents, antibiotic, chemical therapy agents, narcotic, quit smoking aids and the like.

The drug relating to the present invention includes not only drugs in the form of a free base, but also physiologically acceptable salts thereof. While such salt is not particularly limited, examples thereof include, but are not limited to, formate, acetate, lactate, adipate, citrate, tartrate, methanesulfonate, fumarate, maleate and the like, and examples of acid addition salts with inorganic acid include hydrochloride, sulfate, nitrate, phosphate and the like. In the present invention, the drug may be a solvate, a hydrate or a non-hydrate.

The present invention is particularly advantageous for the production of a patch preparation containing a basic drug having a basic group. Examples of the basic drug having a basic group include drugs having one or more functional groups selected from the group consisting of an alcoholic hydroxyl group, a thiol group, a phenolic hydroxyl group, and an amino group (e.g., primary ($-NH_2$), secondary (-NRH), tertiary (-NRR')). That is, when a basic drug and lactic acid as an acidic additive are co-present in an adhesive layer, they react to form a salt, the transdermal absorbability of the drug may decrease. In the present invention, as mentioned below, the opportunity of forming such salt can be reduced, since lactic acid is added to an adhesive layer containing a drug after forming the adhesive layer.

In addition, the present invention is particularly advantageous for the production of a patch preparation containing a solid drug. The solid drug means a drug which is solid at room temperature (25°C), that is, a drug having a melting point of not less than 25°C. The melting point here is a value according to differential scanning calorimetry (DSC). An adhesive layer containing a drug is generally formed by applying a mixture of an adhesive polymer, a drug and the like in a solvent and drying the coated film. However, a solid drug may form a crystal core in the drying step of the coated film. Even if such crystal core is formed, it can be dissolved by adding the below-mentioned lactic acid solution to the adhesive layer, which enables maintenance of the transdermal absorbability of the drug.

The production method of the present invention includes at least the following steps (a) and (b), and the patch preparation obtained thereby has an adhesive layer containing a drug, lactic acid and an organic liquid component. Each step is explained in detail in the following.

[step (a)]

In the production method of the present invention, an adhesive layer containing a drug, an adhesive such as an acrylic polymer and the like and an organic liquid component is first formed. Here, the adhesive layer does not contain lactic acid.

In consideration of the final form of the patch preparation, the adhesive layer is preferably formed on one surface of the support or release liner. In the following, an adhesive layer formed on one surface of the support or release liner is also referred to as "an adhesive sheet".

The adhesive sheet is preferably produced by manufacturing a laminate wherein a support, an adhesive layer and a release liner are formed in this order, and removing the release liner or support from the laminate. Since removal of the release liner is easy, it is particularly preferably produced by removing the release liner from the laminate. The content of each component in the adhesive layer in the following adhesive sheet is shown by a rate (wt%) relative to the total weight of the adhesive layer by assuming the adhesive layer containing lactic acid, namely, the adhesive layer of the patch preparation.

The amount of a drug contained in the adhesive layer is not particularly limited since it needs to be determined according to the age, symptom and the like of the patients who receive the administration. The drug is present in the adhesive layer in an amount sufficient to provide desired results in the treatment of disease, condition or disorder, for example, desired therapeutic effect, which is referred to as an effective amount in the present specification. The effective amount of the drug means, for example, an amount of the drug that provides a concentration of the drug in blood lower than a toxic level and sufficient to provide a selected effect over a predetermined time. While the effective amount varies depending on the area of the patch preparation, it is preferably not less than about 0.1 wt%, more preferably not less than about 0.5 wt%, particularly preferably not less than about 0.8 wt%, relative to the total weight of the adhesive layer. Since an excessive amount may exert an adverse influence on the property of the adhesive layer, the upper limit thereof is preferably not more than about 50 wt%, more preferably not more than about 40 wt%, particularly preferably not more than about 30 wt%.

As an adhesive in the adhesive layer, an acrylic polymer is used. The acrylic polymer is preferably contained at 30 - 80 wt%, more preferably 40 - 70 wt%, relative to the total weight of the adhesive layer.

The acrylic polymer in the present invention is preferably an acrylic polymer containing an alkyl(meth)acrylate unit as the main component (main constituting unit). The acrylic polymer containing an alkyl(meth)acrylate unit as the main component (main constituting unit) is preferably a copolymer of alkyl(meth)acrylate (first monomer component) and a vinyl monomer having a functional group capable of being involved in a crosslinking reaction (second monomer component), and a copolymer wherein other monomer (third monomer component) is further polymerized is particularly preferable in view of the easiness of a crosslinking treatment, adhesiveness to human skin, dissolution property of the drug and the like.

Examples of the above-mentioned alkyl(meth)acrylate (the first monomer component) include alkyl(meth)acrylate wherein the alkyl group is a linear, branched chain or cyclic alkyl group having a carbon number of 1 to 18 (e.g., methyl, ethyl, n-propyl, isopropyl, n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl, n-nonyl, cyclononyl, n-decyl, cyclodecyl, n-undecyl, n-dodecyl, n-tridecyl and the like) and the like, preferably alkyl(meth)acrylate wherein the alkyl group is a linear, branched chain or cyclic alkyl group having a carbon number of 4 to 18 (e.g., n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl, n-nonyl, cyclononyl, n-decyl, cyclodecyl, n-undecyl, n-dodecyl, n-tridecyl and the like). To particularly confer adhesiveness at ambient temperature, use of a monomer component that decreases the glass transition temperature of the polymer is preferable. Thus, alkyl(meth)acrylate wherein the alkyl group is a linear, branched chain or cyclic alkyl group having a carbon number of 4 to 8 (e.g., n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl and the like) is more preferable, and alkyl(meth)acrylate wherein the alkyl group is n-butyl, 2-ethylhexyl or cyclohexyl is particularly preferable.

Particularly preferable specific examples of alkyl(meth)acrylate (first monomer component) include butyl acrylate, 2-ethylhexyl acrylate, 2-ethylhexyl methacrylate, cyclohexyl acrylate, cyclohexyl methacrylate, and 2-ethylhexyl acrylate is most preferable. These alkyl(meth)acrylates (first monomer component) may be used alone or in combination of two or more kinds thereof.

In the above-mentioned vinyl monomer having a functional group capable of being involved in a crosslinking reaction (the second monomer component), examples of the functional group capable of being involved in a crosslinking reaction include a hydroxy group, a carboxyl group, a vinyl group and the like, preferably a hydroxy group and a carboxy group. Specific examples of said monomer (the second monomer component) include hydroxyethyl(meth)acrylate, hydroxypropyl(meth)acrylate, (meth)acrylic acid, itaconic acid, maleic acid, maleic anhydride, methaconic acid, citraconic acid, glutaconic acid and the like. Of these, acrylic acid, methacrylic acid and hydroxyethylacrylate are preferable, and acrylic acid is most preferable, since they are easily available. One or more kinds of these monomers (the second monomer component) can be used in combination.

In addition, the above-mentioned other monomer (the third monomer component) is mainly used for adjusting the cohesive force of the adhesive layer, adjusting solubility and releasability of a drug and the like. Examples of the monomer (the third monomer component) include vinyl esters such as vinyl acetate, vinyl propionate and the like; vinyl ethers such as methyl vinyl ether, ethyl vinyl ether and the like; vinyl amides such as N-vinyl-2-pyrrolidone, N-vinylcaprolactam and the like; alkoxy(meth)acrylates such as methoxyethyl(meth)acrylate, ethoxyethyl(meth)acrylate, tetrahydrofurfuryl(meth)acrylate and the like; hydroxy group-containing monomers such as hydroxypropyl(meth)acrylate, $\alpha$-hydroxymethyl acrylate and the like (since such hydroxy group-containing monomer is used as a third monomer component, it is not involved in the crosslinking reaction); (meth)acrylic acid derivatives having an amide group such as (meth)acrylamide, dimethyl(meth)acrylamide, N-butyl(meth)acrylamide, N-methylol(meth)acrylamide and the like; aminoalkyl(meth)acrylates such as aminoethyl(meth)acrylate, dimethylaminoethyl(meth)acrylate, tert-butylaminoethyl (meth)acrylate and the like; alkoxyalkyleneglycol(meth)acrylates such as methoxyethyleneglycol(meth)acrylate, methoxydiethyleneglycol(meth)acrylate, methoxypolyethylene glycol(meth)acrylate, methoxypolypropyleneglycol(meth)acrylate and the like; (meth)acrylonitrile; sulfo group-containing monomers such as styrene sulfonic acid, ally sulfonic acid, sulfopropyl(meth)acrylate, (meth)acryloyloxynaphthalenesulfonic acid, acrylamidemethylsulfonic acid and the like; vinyl group-containing monomers such as vinyl piperidone, vinyl pyrimidine, vinyl piperazine, vinyl pyrrole, vinyl imidazole, vinyl oxazole, vinyl morpholine etc., and the like. Among these, vinyl esters and vinyl amides are preferable, vinyl ester is preferably vinyl acetate, and vinyl amide is preferably N-vinyl-2-pyrrolidone. One or more kinds of these monomers (the third monomer component) can be used in combination.

When the acrylic polymer is a copolymer of alkyl(meth)acrylate (the first monomer component) and a vinyl monomer having a functional group capable of being involved in a crosslinking reaction (the second monomer component), the copolymerization ratio (first monomer component/second monomer component) is preferably 85 - 99 wt%/1 - 15 wt%, more preferably 90 - 99 wt%/1 - 10 wt%.

When the acrylic polymer is a copolymer of alkyl(meth)acrylate (the first monomer component), a vinyl monomer having a functional group capable of being involved in a crosslinking reaction (the second monomer component), and a monomer other than these (the third monomer component), the copolymerization ratio (first monomer component/ second monomer component/third monomer component) is preferably 40 - 94 wt%/1 - 15 wt%/5 - 50 wt%, more preferably 50 - 89 wt%/1 - 10 wt%/10 - 40 wt%.

While the polymerization reaction may be performed by a method known per se and is not particularly limited, for example, a method including reacting the above-mentioned monomer in a solvent (e.g., ethyl acetate and the like) in the presence of a polymerization initiator (e.g., benzoyl peroxide, azobisisobutyronitrile and the like) at 50 - 70°C for 5 - 48 hr can be mentioned.

The acrylic polymer in the present invention is particularly preferably a 2-ethylhexylacrylate/acrylic acid/N-vinyl-2-pyrrolidone copolymer, a 2-ethylhexylacrylate/2-hydroxyethylacrylate/vinyl acetate copolymer, a 2-ethylhexylacrylate/ acrylic acid copolymer and the like, more preferably a 2-ethylhexylacrylate/acrylic acid/N-vinyl-2-pyrrolidone copolymer.

While the glass transition temperature of the acrylic polymer in the present invention also varies depending on the copolymer composition, it is generally preferably -100 to -10°C, more preferably -90 to -20°C, from the aspect of adhesiveness of a patch preparation.

The adhesive layer contains an organic liquid component. Such organic liquid component can be used without any particularly limitation as long as the component itself is liquid at room temperature (25°C), shows a plasticizing action, and is compatible with the above-mentioned acrylic polymer.

The organic liquid component softens the adhesive layer, and reduces physical irritation to the skin due to the patch preparation. Specific examples of the organic liquid component include fatty acid ester (hereinafter to be also abbreviated as "C8 - 18 (12 - 16)-C1 - 18 fatty acid ester") such as isopropyl myristate, ethyl laurate, isopropyl palmitate, ethyl oleate, isostearyl laurate, isotridecyl myristate, octyl palmitate and the like, which is formed from a fatty acid having a carbon number of 8 to 18 (preferably 12 - 16) and a monohydric alcohol having a carbon number of 1 to 18; fatty acid having a carbon number of 8 to 9 [for example, caprylic acid (octanoic acid, C8), pelargonic acid (nonanoic acid, C9) and the like]; glycerin fatty acid ester; glycols such as ethylene glycol, diethylene glycol, triethylene glycol, polyethylene glycol, 1,3-propanediol, polypropylene glycol and the like; fats and oils such as olive oil, castor oil, squalene and the like; organic solvent such as dimethyl sulfoxide, dimethylformamide, dimethylacetamide, dimethyllauryl amide, dodecyl pyrrolidone, isosorbitol, oleyl alcohol, lauric acid, N-methyl-2-pyrrolidone and the like; liquid surfactant such as polyoxyethylene alkyl ether sodium sulfate, polyoxyethylene lauryl ether sodium sulfate, sodium alkylnaphthalenesulfonate, polyoxyethyleneoleyl amine, polyoxyethylene oleyl ether sodium phosphate, polyoxyl stearate, decaglyceryl laurate, polyoxyethylene sorbitan monolaurate, polyoxyethylene sorbitan monostearate, sorbitan monolaurate, sorbitan trioleate, polyoxyethylenesorbitol tetraoleate, glycerol monooleate, sucrose ester of fatty acid, tocopherol and the like; hydrocarbons; plasticizer conventionally known such as phthalic acid ester, and the like; ethoxylated stearyl alcohol; glycerol and the like. One kind alone or two or more kinds of these organic liquid components are used in combination. Among these, C8 - 18 (12-16)-C1 - 18 fatty acid ester is preferable, and isopropyl myristate is particularly preferable.

The content of the organic liquid component in the adhesive layer is preferably 5 - 60 wt%, more preferably 10 - 50 wt%, of the total weight of the adhesive layer. When the content is less than 5 wt%, the adhesive layer may not be plasticized sufficiently, a good soft feeling may not be obtained, or skin irritation may not be decreased sufficiently. Conversely, when it exceeds 60 wt%, the organic liquid component cannot be maintained in the adhesive even by the cohesive force possessed by the adhesive, it causes blooming on the surface of the adhesive layer, thus resulting in too weak adhesive force, which in turn possibly causes falling off of the preparation from the skin surface during use.

When desired, physical crosslinking by radiation such as UV irradiation, electron beam irradiation and the like, or a chemical crosslinking treatment using various crosslinking agents may be applied to the adhesive layer. What is called a gel-like adhesive layer, wherein an adhesive layer containing the above-mentioned acrylic polymer and an organic liquid component is crosslinked, is preferable, since it confers a soft feeling to the skin as well as has appropriate adhesiveness and cohesive force. While the crosslinking agent for applying a crosslinking treatment to an adhesive layer of the adhesive sheet is not particularly limited, examples thereof include isocyanate compounds, organometallic compounds (e.g., zinc zirconium, zinc alaninate, zinc acetate, zinc glycine ammonium compounds, and the like), metal alcoholate (e.g., tetraethyl titanate, tetraisopropyl titanate, aluminum isopropylate, aluminum sec-butyrate and the like), metal chelate compounds (e.g., dipropoxybis(acetylacetonate)titanium, tetraoctylene glycol titanium, aluminum isopropylate, ethyl acetoacetate aluminum diisopropylate, aluminum tris (ethyl acetoacetate), aluminum tris (acetylacetonate) and the like), and the like. Particularly, when the adhesive layer of the adhesive sheet contains an isocyanate compound, a decrease in the cohesive force of the adhesive layer during adhesion of the patch preparation to the human skin can be reduced, and cohesive failure does not easily occur during detachment of the adhesive layer. Thus, an isocyanate compound is preferable. According to the method of the present invention, a sufficiently high crosslinking structure can be formed due to the absence of lactic acid, which exerts an adverse influence on the crosslinking reaction, in the adhesive layer, a patch preparation having a high holding power can be produced. An adhesive layer crosslinked by an isocyanate compound shows a particularly high holding power (cohesive force after adhesion to the skin).

Examples of the isocyanate compound include aliphatic diisocyanate such as tetramethylene diisocyanate, hexamethylene diisocyanate and the like, alicyclic diisocyanate such as isophorone diisocyanate, hydrogenated xylylene diisocyanate, hydrogenated toluene diisocyanate, hydrogenated diphenylmethane diisocyanate and the like, aromatic aliphatic diisocyanate such as xylylene diisocyanate and the like, aromatic diisocyanate such as tolylenediisocyanate, 4,4'-diphenylmethane diisocyanate etc., and the like. The above-mentioned isocyanate compound may be used alone, or may be used in combination with other crosslinking agent.

One or more kinds of the above-mentioned crosslinking agents may be used in combination for the crosslinking treatment. While the amount of the crosslinking agent varies depending on the kind of the crosslinking agent and the adhesive (acrylic polymer), in general, it is preferably 0.01 - 10 wt%, more preferably 0.05 - 5 wt%, particularly preferably 0.1 - 0.3 wt%, relative to the total weight of the adhesive layer of the patch preparation. When it is less than 0.01 wt%, a sufficient cohesive force cannot be conferred to an adhesive layer since the crosslinking points are too few, which in turn may result in adhesive residue and strong skin irritation caused by cohesive failure during the detachment of the preparation from the skin. When it is more than 10 wt%, sufficient skin adhesion may not be afforded, though the cohesive force is high. In addition, skin irritation may occur due to the residual unreacted crosslinking agent. Since the effect of increasing the cohesive force of the adhesive layer, which is achieved by adding lactic acid to an adhesive layer formed without containing lactic acid, becomes more remarkable, the amount of the crosslinking agent is preferably 0.03 - 0.6 part by weight, more preferably 0.05 - 0.5 part by weight, still more preferably 0.15 - 0.5 part by weight, most preferably 0.15 - 0.35 part by weight, relative to 100 parts by weight of the acrylic polymer in the adhesive layer. The chemical crosslinking treatment may be performed by, e.g., adding a crosslinking agent, followed by heating the adhesive layer at a crosslinking reaction temperature or higher and storing thereof, that is, an aging step. The heating temperature which may be chosen depending on the kind of the crosslinking agent is preferably 60-90°C, more preferably 60-80°C. A time for the heating is preferably 12-96 hours, more preferably 24-72 hours.

The adhesive layer may be formed on one surface of the support or release liner. The adhesive layer contains a drug, an acrylic polymer, an organic liquid component and a crosslinking agent etc. (crosslinking agent is used to form a crosslinked adhesive layer), and can contain a flavor, a colorant, and other additives.

In addition, the adhesive layer is preferably a non-aqueous adhesive layer. The non-aqueous adhesive layer here is not necessarily limited to one completely free of water, but includes those containing a slight amount of water (e.g., less than 1 wt% of the total weight of an adhesive layer) derived from humidity in the air, skin and the like.

While the support in the adhesive sheet is not particularly limited, preferred is one that does not allow a drug and an organic liquid component in the adhesive layer to pass through the support and be lost from the back face, which decreases their contents (namely, a material impermeable to the organic liquid component and drug).

Specific examples include a single film of polyester (e.g., polyethylene terephthalate etc.), nylon, polyvinyl chloride, polyethylene, polypropylene, ethylene-vinyl acetate copolymer, polytetrafluoroethylene, ionomer resin and the like, a metal foil, and a laminate film wherein two or more kinds of films selected therefrom are laminated and the like. Of these, to improve adhesiveness (anchor property) of a support to an adhesive layer, it is preferable to use, as a support, a laminate film of a non-porous film made from the above-mentioned material and the following porous film, and form the adhesive layer on the porous film side. The thickness of the non-porous film is preferably 2 - 100 $\mu$m, more preferably 2-50 $\mu$m.

The porous film is not particularly limited as long as the anchor property to an adhesive layer is improved and, for example, paper, woven fabric, non-woven fabric (e.g., polyester (e.g., polyethylene terephthalate and the like) non-woven fabric and the like), the above-mentioned film (e.g., a single film of polyester, nylon, Saran (trade name), polyethylene, polypropylene, ethylene-vinyl acetate copolymer, polyvinyl chloride, ethylene-ethyl acrylate copolymer, polytetrafluoroethylene, metal foil, and the like, and a laminate film wherein two or more kinds of films selected therefrom are laminated and the like), which is mechanically perforated, and the like can be mentioned. Particularly, paper, woven fabric and non-woven fabric (e.g., polyester (e.g., polyethylene terephthalate and the like) non-woven fabric and the like) are preferable from the aspects of flexibility of the support. For example, when the porous film is paper, woven fabric, non-woven fabric etc., the fabric weight is preferably 5 - 30 g/m$^2$ to improve anchor property.

The laminate film as a support is produced by a known production method of a laminate film such as dry lamination method, wet lamination method, extrusion lamination method, hot melt lamination method, coextrusion lamination method and the like.

The thickness of the support in the adhesive sheet is not particularly limited but preferably 2-200 μm, more preferably 10-50 μm. When it is less than 2 μm, the handling property such as self-supporting property may become worse. When the thickness is more than 200 μm, the followability may become worse to cause skin discomfort.

The release liner in the adhesive sheet is not particularly limited, and a known release liner can be used. Specific examples thereof include a release liner wherein a release treating agent layer comprised of the release treating agent is formed on the surface of a substrate for a release liner, a plastic film having high releasability by itself, a release liner wherein a release layer comprised of the aforementioned plastic film material having high releasability is formed on the surface of a substrate for a release liner and the like. The release surface of the release liner may be only one surface or both surfaces of the substrate.

In such release liner, the release treating agent is not particularly limited and, for example, release agents such as a long chain alkyl group-containing polymer, a silicone polymer (silicone release agent), a fluorine polymer (fluorine release agent) and the like can be mentioned. Examples of the substrate for a release liner include plastic films such as a polyester (e.g., polyethylene terephthalate etc.) film, a polyimide film, a polypropylene film, a polyethylene film, a polycarbonate film, and the like, and metallized plastic films  wherein a metal is evaporated on these films; papers such as Japanese paper, Western paper, craft paper, glassine paper, fine paper and the like; a substrate made of a fibrous material such as non-woven fabric, cloth and the like; a metal foil and the like.

As the plastic film having high releasability by itself, polyethylene (low density polyethylene, linear low density polyethylene etc.), polypropylene, ethylene-α-olefin copolymers (block copolymer or random copolymer) such as ethylene-propylene copolymer and the like, a polyolefin film made of a mixture of two or more kinds selected from these; polytetrafluoroethylene (Teflon (registered trade mark)) film and the like can be used.

The release liner having a release layer made from a material of a plastic film having high detachability can be formed by laminating or coating the aforementioned plastic film material having high releasability on the aforementioned substrate for a release liner.

While the thickness (total thickness) of the release liner in an adhesive sheet is not particularly limited, it is generally not more than 200 μm, preferably 25 - 100 μm.

While the production method of the adhesive sheet is not particularly limited, the following method is preferable.

An adhesive polymer, a drug and an organic liquid component are added, together with other additives to be added as necessary, to a suitable solvent and the mixture is sufficiently mixed until it becomes homogeneous. Examples of the solvent include ethyl acetate, toluene, hexane, 2-propanol, methanol, ethanol and the like. When a crosslinking agent is added to the adhesive layer, it is added to the mixture and  the mixture is sufficiently mixed. Where necessary, a solvent may be added along with a crosslinking agent and they are mixed.

Then, the obtained mixture is applied to one surface of the support or a release treating surface of the release liner, and dried to form an adhesive layer. The aforementioned application can be performed by, for example, casting, printing and other techniques known per se to those of ordinary skill in the art. Thereafter, a release liner or support is adhered to the adhesive layer to form a laminate. When a crosslinking treatment of the adhesive layer is performed, the release liner or support is adhered to the adhesive layer, and they are left standing at 60 - 90°C, preferably 60 - 70°C, for 24 - 48 hr to promote the crosslinking reaction, whereby a crosslinked adhesive layer is formed.

An adhesive sheet wherein an adhesive layer is formed on one surface of the support or release liner is obtained by removing the release liner or support from the thus-obtained laminate consisting of the support, the adhesive layer and the release liner.

Since the release liner is removed more easily than the support, it is preferable from the aspects of workability to obtain an adhesive sheet, wherein an adhesive layer is formed on one surface of the support, by removing the release liner.

While the thickness of the adhesive layer is not particularly limited, it is preferably 20-300 μm, more preferably 30-300 μm, most preferably 50-300 μm. When the thickness is smaller than 20 μm, it may be difficult to afford a sufficient adhesiveness and to contain an effective amount of a drug. When the thickness is higher than 300 μm, the  formation of the adhesive layer may be difficult (difficult coating).

[step (b)]

In this step, lactic acid is added to the adhesive layer containing a drug, an adhesive such as an acrylic polymer and the like and an organic liquid component, which is formed in the above-mentioned step (a).

The lactic acid used in the present invention may be DL-lactic acid which is a racemate, or L-lactic acid or D-lactic acid which is an optically active form. From the aspect of easy flowability, DL-lactic acid is preferable.

While a method of containing lactic acid in an adhesive layer is not particularly limited, a method including preparing a lactic acid solution wherein lactic acid is dissolved in an organic solvent, and impregnating an adhesive layer with the lactic acid solution is preferable. An adhesive layer is impregnated with the lactic acid solution by using a known application method (application apparatus) such as casting the lactic acid solution on an adhesive surface of the adhesive layer, spin coating, spray coating, brush coating, slot die coating, ink jet coating and the like. Such application of the lactic acid solution to an adhesive surface of the adhesive layer is generally performed at room temperature. While the organic solvent to be used for the lactic acid solution is not particularly limited as long as it can dissolve lactic acid, from the aspects of penetratability of lactic acid into the adhesive layer, for example, an organic solvent having 2 - 4 carbon atoms (total carbon number also including carbon atom of polar group) and having a polar group such as carbonyl group [-C(O)-], ester group [-C(O)-O-], carboxy group [-COOH] and hydroxy group [-OH] and the like is preferable. Specific preferable examples thereof include ethyl acetate, ethyl alcohol, acetone, acetaldehyde and the like. Of these, ethyl acetate is particularly preferable. While the concentration of lactic acid in the lactic acid solution is not particularly limited, it is preferably about 5 - 50 wt%.

The lactic acid solution can be penetrated into the adhesive layer basically by simply leaving after application of the lactic acid solution to an adhesive surface of the adhesive layer. Preferably, the temperature of the environment during leaving is set to 5 - 40°C (preferably 15 - 30°C), and the temperature is preferably maintained for about 10 sec - 10 min (preferably 30 sec - 5 min). In this way, the lactic acid solution is rapidly penetrated into the adhesive layer to efficiently impregnate the adhesive layer with the lactic acid solution.

After impregnating the adhesive layer with the lactic acid solution, the organic solvent derived from the lactic acid solution in the adhesive layer is evaporated to produce the final patch preparation (step (c)).

The evaporation of the organic solvent means evaporating the organic solvent under heating, wherein the lower limit of the heating temperature exceeds 40°C and the upper limit is not more than 100°C, preferably 60 - 90°C, and the heating time is preferably about 30 sec - 5 min.

After evaporation of the organic solvent, the patch preparation is completed by newly laminating a release liner on an adhesive surface of the adhesive layer when the adhesive sheet is a laminate of the adhesive layer and the support, or by newly laminating a support on an adhesive surface of the adhesive layer when the adhesive sheet is a laminate of the adhesive layer and the release liner. Specific examples of the support to be newly laminated and the release liner to be newly laminated here include those similar to the specific examples of the support and release liner recited in the explanation of the aforementioned [step (a)].

In the thus-obtained patch preparation, the content of the lactic acid in the adhesive layer is preferably 0.1 - 13 wt%, more preferably 1 - 10 wt%, most preferably 1 - 8 wt%, relative to the total weight of the adhesive layer. Therefore, the concentration and the amount of the lactic acid solution to be applied to a surface of the adhesive layer are determined to achieve such lactic acid content. When the lactic acid content of the adhesive layer in the patch preparation is too low, a drug permeation-promoting effect (transdermal absorption-promoting effect) into the skin cannot be sufficiently exhibited. When it is too high, skin irritation may become stronger. The organic liquid component contained in the adhesive layer in step (a) may partially evaporate in step (c). In such a case, the organic liquid component in an amount comparable to the evaporation amount can be contained in advance in the adhesive layer in step (a). Examples

The present invention is explained in detail in the following by referring to Examples, which are not to be construed as limitative. In the following sentences, "parts" and "%" all mean "parts by weight" and "wt%", respectively.

[Preparation of acrylic polymer A]

Under an inert gas atmosphere, 2-ethylhexylacrylate (75 parts), N-vinyl-2-pyrrolidone (22 parts), acrylic acid (3 parts) and azobisisobutyronitrile (0.2 part) were subjected to solution polymerization in ethyl acetate at 60°C to give an acrylic copolymer (acrylic polymer A) solution. The glass transition point of the acrylic copolymer (acrylic polymer A) was -45.2°C.

[Example 1]

A solution of acrylic polymer A (53.8 parts), lidocain (hereinafter to be referred to as "LDC") (6.0 parts), and isopropyl myristate (hereinafter to be referred to as "IPM") (30.0 parts) in a moderate amount of ethyl acetate was sufficiently mixed until it became homogeneous. As a crosslinking agent, trifunctional isocyanate (CORONATE HL (manufactured by Japan Polyurethane Industry), 0.2 part) was added. The mixture was sufficiently mixed and stirred until it became homogeneous to give a coating solution. The obtained coating solution was applied to the release-treated one surface of a release liner, which was a 75 $\mu$m-thick PET film subjected to a release treatment, such that the thickness of the plaster layer after drying was about 60 $\mu$m, and dried to form an adhesive layer. The adhesive surface of the adhesive layer thus formed was adhered to a nonwoven fabric side of a laminate film of a 3.5 $\mu$m-thick PET film and a PET nonwoven fabric with a fabric weight of 12 g/m$^2$ to give a laminate. The laminate was left standing at 70°C for 48 hr to prepare a crosslinked adhesive layer. The melting point of lidocain was 66 - 69°C. The melting point was measured by a DSC apparatus (manufactured by Seiko Instruments Inc. (SII), model number DSC6220).

The above-mentioned release liner was detached to give an adhesive sheet having a crosslinked adhesive layer on one surface of a support. A solution of DL-lactic acid in ethyl acetate (lactic acid:ethyl acetate = 1:2 (weight ratio)) was applied to an adhesive surface of the adhesive layer by slot die application, the layer was maintained at 23°C for 3 min, and dried at 80°C for 3 min such that the an lactic acid content of the adhesive layer after drying was 10 parts relative to the crosslinked adhesive layer (90 parts). After drying, a peel-treated release liner was separately prepared, an adhesive surface of the adhesive layer was adhered to the peel-treated surface of the release liner to give the patch preparation of Example 1.

[Examples 2 - 4]

In the same manner as in Example 1 except that the blending amounts of Table 1 below were adopted, the patch preparations of Examples 2 - 4 were obtained.

[Comparative Example 1]

A solution of acrylic polymer A (53.8 parts), lidocain (6.0 parts), and IPM (30.0 parts) in a moderate amount of ethyl acetate was sufficiently mixed until it became homogeneous. As a crosslinking agent, trifunctional isocyanate (CORONATE HL (manufactured by Japan Polyurethane Industry), 0.2 part) and lactic acid (10 parts) were added. The mixture was sufficiently mixed and stirred until it became homogeneous to give a coating solution. The obtained coating solution was applied to the release-treated one surface of a release liner, which was a 75 $\mu$m-thick PET film subjected to a release treatment, such that the thickness of the adhesive layer after drying was about 60 $\mu$m, and dried to form an adhesive layer. The adhesive surface of the adhesive layer thus formed was adhered to a nonwoven fabric side of a laminate film of a 3.5 $\mu$m-thick PET film and a PET nonwoven fabric with a fabric weight of 12 g/m$^2$ to give a laminate. The laminate was left standing at 70°C for 48 hr to give the patch preparation of Comparative Example 1.

[Comparative Examples 2 - 4]

In the same manner as in Comparative Example 1 except that the blending amounts of Table 1 below were adopted, the patch preparations of Comparative Examples 2 - 4 were obtained.

<Experimental Example>

[Holding power]

A sample is cut in width 10 mm, length 50 mm and one end (about 25 mm) thereof is pressed against a bakelite (phenol resin) plate by one reciprocation of a roller (weight 850 g). The other end is reinforced with an auxiliary sheet. This is set on a hook in an apparatus stabilized at a temperature of 40±2°C, left for 30 min, attached with a load (300 g) and left until natural falling occurs. The retention time then was measured. The experiment was performed at n=3, and the total 3 points were averaged.

[Gel fraction]

A patch preparation was cut into 10 cm$^2$ and the weight ($W_1$) of the adhesive layer of the sample was measured. Then, the sample was adhered to a polytetrafluoroethylene (PTFE) porous membrane (NTF film manufactured by NITTO DENKO CORPORATION), and the sample was immersed in 100 ml of ethyl acetate for 24 hr, and ethyl acetate was exchanged. This operation was repeated 3 times to extract the solvent soluble content. Then, the sample was taken out, dried and the weight ($W_2$) of the adhesive layer was measured. The gel fraction was calculated by the following formula.

$$gel\ fraction\ (\%) = (W_2 \times 100) / (W_1 \times A/B)$$

A= weight of (an adhesive + a crosslinking agent)
B= weight of (an adhesive + organic liquid component + crosslinking agent)

The results are shown in Table 1.
Table 1

|  | acrylic polymer A | IPM | LDC | lactic acid | crosslinking agent | thickness of adhesive laver | gel fraction (%) | holding power (min) |
|---|---|---|---|---|---|---|---|---|
| Ex. 1 | 53.8 | 30.0% | 6.0% | 10.0% | 0.2% | 60 μm | 56 | 1.3 |
| Ex. 2 | 57.8 | 30.0% | 6.0% | 6.0% | 0.2% | 60 μm | 51 | 25.2 |
| Ex. 3 | 60.8 | 30.0% | 6.0% | 3.0% | 0.2% | 60 μm | 48 | 32.0 |
| Ex. 4 | 60.9 | 30.0% | 6.0% | 3.0% | 0.1% | 60 μm | 36 | 5.6 |
| Comp. Ex. 1 | 53.8 | 30.0% | 6.0% | 10.0% | 0.2% | 60 μm | 14 | 0.4 |
| Comp. Ex. 2 | 57.8 | 30.0% | 6.0% | 6.0% | 0.2% | 60 μm | 9 | 0.4 |
| Comp. Ex. 3 | 60.8 | 30.0% | 6.0% | 3.0% | 0.2% | 60 μm | 10 | 0.5 |
| Comp. Ex. 4 | 60.9 | 30.0% | 6.0% | 3.0% | 0.1% | 60 μm | 5 | 0.5 |

As is clear from the results of Table 1, the preparations of Examples 1 - 4 obtained by adding lactic acid to the adhesive layer without containing lactic acid showed high gel fraction as compared to the preparations of Comparative Examples 1 - 4 obtained by mixing lactic acid with the adhesive layer together with an acrylic polymer, a drug and the like, and the adhesive layer was sufficiently crosslinked. The preparations of Examples 1 - 4 showed high holding power and high cohesive force of the adhesive layer as compared to the preparations of Comparative Examples 1 - 4.
In addition, the effect of increasing the cohesive force of the adhesive layer by adding lactic acid to the adhesive layer after formation of the adhesive layer without containing lactic acid was particularly remarkable when the lactic acid content of the adhesive layer was 3 - 6 wt%.
From the results of Examples 3, 4 and Comparative Examples 3, 4, moreover, it is clear that the effect of the present invention, namely, the effect of increasing the cohesive force of the adhesive layer, which is achieved by adding lactic acid to an adhesive layer without containing lactic acid, is remarkable when the amount of the crosslinking agent added is 0.16 part by weight - 0.32 part by weight relative to 100 parts by weight of the acrylic polymer.

Industrial Applicability

According to the production method of the present invention, since lactic acid is later added to an adhesive layer without containing lactic acid, an influence of lactic acid on the property of the adhesive layer can be reduced. Particularly, when a patch preparation containing a drug and lactic acid in a crosslinked adhesive layer is produced, since an adverse influence of lactic acid on the crosslinking reaction of the adhesive layer can be suppressed, a patch preparation showing an improved holding power after adhesion to the skin (i.e., cohesive force of adhesive layer after adhesion), while maintaining a transdermal drug absorption-promoting effect of lactic acid, can be produced.
This application is based on a patent application No. 2011-021203 filed in Japan, the contents of which are incorporated in full herein.

**Claims**

1. A method of producing a patch preparation comprising, in an adhesive layer, a drug (excluding 2-(4-ethyl-1-piperazinyl)-4-(4-fluorophenyl)-5,6,7,8,9,10-hexahydrocycloocta[b]pyridine and a physiologically acceptable salt thereof), and lactic acid, comprising

   (a): a step of forming an adhesive layer containing a drug, an acrylic polymer and an organic liquid component compatible with the acrylic polymer, but without containing lactic acid, and
   (b): a step of adding lactic acid to said adhesive layer.

2. The method according to claim 1, wherein a crosslinked adhesive layer is formed in step (a).

3. The method according to claim 1 or 2, wherein the adhesive layer is formed on one surface of a support or a release liner in step (a).

4. The method according to any one of claims 1 to 3, wherein the lactic acid is contained in the adhesive layer by immersing the adhesive layer in a lactic acid solution obtained by dissolving lactic acid in an organic solvent in step (b).

5. The method according to claim 4, wherein the adhesive layer is impregnated with the lactic acid solution by applying the lactic acid solution to a surface of the adhesive layer and leaving the adhesive layer at 5 - 40°C for a given time.

6. The method according to claim 4 or 5, further comprising, after step (b), (c): a step of evaporating an organic solvent derived from the lactic acid solution contained in the adhesive layer.

## INTERNATIONAL SEARCH REPORT

| | International application No. |
|---|---|
| | PCT/JP2012/052309 |

**A. CLASSIFICATION OF SUBJECT MATTER**
*A61K47/12*(2006.01)i, *A61K9/70*(2006.01)i, *A61K31/167*(2006.01)i, *A61K47/32* (2006.01)i

According to International Patent Classification (IPC) or to both national classification and IPC

**B. FIELDS SEARCHED**

Minimum documentation searched (classification system followed by classification symbols)
A61K47/12, A61K9/70, A61K31/167, A61K47/32

Documentation searched other than minimum documentation to the extent that such documents are included in the fields searched
Jitsuyo Shinan Koho          1922-1996    Jitsuyo Shinan Toroku Koho   1996-2012
Kokai Jitsuyo Shinan Koho    1971-2012    Toroku Jitsuyo Shinan Koho   1994-2012

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 5-132418 A  (Lintec Corp.), 28 May 1993 (28.05.1993), claims 1, 2; paragraphs [0006], [0007], [0011] to [0026] (Family: none) | 1-6 |
| A | WO 2005/115355 A1  (Hisamitsu Pharmaceutical Co., Inc.), 08 December 2005 (08.12.2005), claims 1, 10; paragraph [0025] & US 2008/0038328 A1 | 1-6 |

| ☒ | Further documents are listed in the continuation of Box C. | ☐ | See patent family annex. |
|---|---|---|---|

\*     Special categories of cited documents:
"A"   document defining the general state of the art which is not considered to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
|---|---|
| 26 March, 2012 (26.03.12) | 03 April, 2012 (03.04.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/052309

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/142295 A1 (Dainippon Sumitomo Pharma Co., Ltd.), 13 December 2007 (13.12.2007), claims 1 to 6; paragraph [0048]; examples 34, 44 to 63 & US 2009/0169605 A1 & EP 2036559 A1 | 1-6 |
| A | WO 96/16642 A1 (Hisamitsu Pharmaceutical Co., Inc.), 06 June 1996 (06.06.1996), entire text & US 5866157 A & EP 788792 A1 | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007142295 A
- WO 9616642 A

- JP 2011021203 A